Europäisches Patentamt

(19) European Patent Office

Office européen des brevets

(11) Publication number: **0 460 831 A2**

# (12) EUROPEAN PATENT APPLICATION

(21) Application number: 91304656.1

(22) Date of filing: 22.05.91

(51) Int. Cl.⁵: **C07C 67/00, C07C 69/68**

(30) Priority: 08.06.90 GB 9012813

(43) Date of publication of application:
11.12.91 Bulletin 91/50

(84) Designated Contracting States:
AT BE CH DE DK ES FR GB IT LI NL SE

(71) Applicant: BP Chemicals Limited
Belgrave House 76 Buckingham Palace Road
London, SW1W 0SU (GB)

(72) Inventor: Kelly, Raymond Leslie, BP
Chemicals Ltd.
Salt End
Hedon, Hull HU12 8DS (GB)

(74) Representative: Krishnan, Suryanarayana
Kalyana et al
BP INTERNATIONAL LIMITED Patents &
Agreements Division Chertsey Road
Sunbury-on-Thames Middlesex TW16 7LN
(GB)

(54) Production of hydroxy carboxylic compounds.

(57) This invention relates to a process for the production of alpha-hydroxy carboxylic compounds such as lactic acid or its ester by reacting a carbonyl compound having at least one hydroxyl group alpha to the carbonyl function with a hydroxy compound of the formula $R^3OH$ at elevated temperature in the presence of a transition metal compound as catalyst. Lactic acid and lactates produced by this process are items of commerce and are useful as solvents and chemical intermediates.

EP 0 460 831 A2

This invention relates to a process for the production of alpha-hydroxy carboxylic compounds from alpha-hydroxy carbonyl compounds by reaction of the latter with an alcohol in the presence of a catalyst.

It is known to produce alpha-hydroxy carboxylic acid salts from alpha-hydroxy carbonyl compounds in the presence of an alkali hydroxide. A specific example of such a process is the production of alkali metal lactates from dihydroxyacetone.

Thus

$$HO.H_2C.CO.CH_2OH \xrightarrow{\quad NaOH \quad} H_3C.CH(OH).COONa$$

The disadvantage of this process is that the yields are relatively low and that further acidification is required to convert the lactate salts to the corresponding free lactic acid, a very desirable commodity. However, in such a process for each equivalent of lactic acid produced one equivalent of an acid and one equivalent of a base are consumed thus rendering the process commercially non-viable.

It has now been found such hydroxy-carboxylic compounds can be produced by catalytic reaction of an alpha-hydroxy carbonyl compound with an alcohol in the presence of a catalyst.

Accordingly, the present invention is a process for the production of alpha-hydroxy carboxylic compounds said process comprising reacting

(a) a carbonyl compound having at least one hydroxyl group alpha to the carbonyl function with

(b) a hydroxy compound of the formula $R^3OH$ wherein $R^3$ is an alkyl group, an alkenyl group, a cycloalkyl group or an aryl group

at elevated temperature in the presence of a transition metal compound as catalyst.

By the term "alpha-hydroxy carboxylic compound" is meant here and throughout the specification that the product of the reaction is primarily lactic acid, an alpha-hydroxy carboxylic acid ester or mixtures thereof. The nature of the product would depend upon the nature of reactant (b).

The carbonyl compounds having at least one hydroxyl group alpha to the carbonyl function and used as reactant (a) in the present invention have the generic formula:

$$HO.H_2C.CO.C(R)(R^1)(R^2) \qquad (I)$$

wherein each of $R$, $R^1$ and $R^2$ may be the same or different groups selected from hydrogen, hydroxyl, alkyl, cycloalkyl, alkaryl, aryl, aralkyl, halo, nitro, nitrile and a sugar residue.

One of the groups $R$, $R^1$ and $R^2$ is most preferably a hydroxyl group. In such a case, the reactant (a) preferably has the generic formula:

$$HOH_2C.CO.C(OH)(R^1)(R^2) \qquad (II)$$

where $R^1$ and $R^2$ have the same significance as in formula (I) above.

Where $R$ or $R^1$ or $R^2$:

(i) or all three taken together represent an alkyl group, it is a straight or branched $C_1$-$C_{10}$ alkyl group;

(ii) or all three taken together represent an aryl group, it has one or more aromatic nuclei whether or not forming fused ring structures;

(iii) represent a halo group, it is a chloro or a bromo group;

or, (iv) represent a sugar residue, such residues being those derived from glucose, fructose or sucrose.

A specific but simple example of compounds of this type is 1,3-dihydroxyacetone represented by $HOH_2C.CO.CH_2OH$ and in the following discussion this compound will hereinafter be denoted as "DHA" and will be used particularly as an example to illustrate the process of the present invention.

As for the reactant (b), it has a generic formula:

$$R^3OH \qquad (III)$$

wherein $R^3$ is H, an alkyl, alkenyl, cycloalkyl or an aryl group.

The group $R^3$ is preferably a $C_1$-$C_{10}$ hydrocarbyl group. Where $R^3$ is a cycloalkyl group, it is preferably a cyclohexyl group.

The reaction is suitably carried out under homogeneous conditions and under these conditions, it will be understood that the transition metal compound used as catalyst will be soluble in the reaction system.

The catalyst for the reaction is a transition metal compound derived from a transition metal belonging to any one of Groups III-VIII of the Periodic Table of Elements appearing on pages 448-449 of the Handbook of Chemistry & Physics Ed. by C D Hodgman et al, 44th Edition and published by The Chemical Rubber Publishing Co. (1963). Thus the compound used as catalyst may be for example that of titanium, zirconium, hafnium, vanadium, niobium, chromium, ruthenium or rhodium. It is preferable that the transition metal compounds used as catalyst are soluble in the reaction system. Thus, the compounds may be selected from halides, nitrates, sulphates, alkoxides, oxides, carboxylates, acetylacetonates and cyclopentadienyl complexes or derivatives thereof of the metals.

The amount of catalyst used is suitably from 0.001 to 1 mole, preferably from 0.01 to 0.2 moles based on the weight of reactant (a).

The reaction is preferably carried out under reflux conditions. Thus the reaction temperature would depend upon the boiling points of the reactants (a) and (b).

The reaction can be carried out at ambient or elevated pressures.

It is preferable to remove as much of the water as is possible from the reaction system during the reaction. Water removal may be achieved by conventional means such as e.g. a Dean and Stark apparatus.

It is preferable to carry out the reaction under an atmosphere inert under the reaction conditions such as e.g. nitrogen.

In some cases, depending upon the nature of reactants (a) and (b) it may be necessary to add a liquid diluent which is inert under the reaction conditions to reduce the viscosity of the reaction system. An example of such a diluent is diethylene glycol dimethyl ether. Such diluents enable the reaction to proceed at a more uniform rate to completion.

The reaction can be carried out batchwise or continuously.

The product of the reaction according to the present invention is an alpha-hydroxy carboxylic acid ester of the formula:

$$R^3OOC.CH(OH).C(R)(R^1)(R^2) \qquad (IV)$$

As is evident from the above formula, when the reaction product is an ester, the group $R^3$ esterifying the carboxylic acid is derived from the alkyl, alkenyl, cycloalkyl or aryl residue of the hydroxylic reactant (b). Thus, the product of the reaction of DHA with, for example, n-butanol in the presence of titanium isopropoxide or titanium lactate catalyst would be n-butyl lactate. In the same reaction, if $R^3$ = H, the reaction product would correspondingly be lactic acid.

The process of the present invention is particularly suited to producing lactate esters. However, due to the inevitable presence of by-product water formed during such a reaction, the products may contain minor amounts of the free alpha-hydroxy carboxylic acid corresponding to the ester. This free acid in this case is probably formed due to the hydrolysis of the ester product in situ in the presence of water under the reaction conditions.

Lactate esters are items of commerce in their own right for uses such as solvents and as chemical intermediates. They can, however, also be hydrolysed to lactic acid by conventional means.

The present inventions is further illustrated with reference to the following Examples illustrated using DHA.

## Examples

Note: Dihydroxyacetone is commercially available as the dimer which is readily dissociated in solution to the monomeric form. Diethylene glycol dimethyl ether was present as an internal standard for analysis by gas chromatography. Tilcom TL is an aqueous solution of titanium lactate and a sample was provided by Tioxide for evaluation.

## Example 1

Into a 250ml round-bottomed flask equipped with side arm was added 10g of dihydroxyacetone dimer, 150ml of n-butanol and 1g of diethylene glycol dimethyl ether. The flask was also equipped with an 8 plate Oldershaw distillation column surmounted by Dean and Stark apparatus, water cooled condenser and nitrogen blanket. Water was removed by refluxing the mixture in the flask and removing the lower aqueous layer from the Dean and Stark apparatus. Initially the water content of the mixture in the flask, due to associated water in dihydroxyacetone dimer and n-butanol, as measured by Karl Fisher analysis was 1.7%. When the water content was 0.01%, 2ml of titanium (IV) isopropoxide was added and an exothermic reaction took place with a colour change to orange. An immediate analysis showed a 17.2% yield of n-butyl lactate based on dihydroxyacetone and after a further 14 hours heating under reflux this had increased to 20.1%.

## Example 2

Into a 250ml round-bottomed flask equipped with side-arm, 20 plate Oldershaw column surmounted by Dean and Stark apparatus, water cooled condenser and nitrogen blanket was added 9g of dihydroxyacetone dimer, 150ml n-butanol, 1g diethylene glycol dimethyl ether and 2ml of aqueous titanium lactate solution (Tilcom TL ex Tioxide). The reaction mixture was heated under reflux for 2.5 hours and water removed by means of the Dean and Stark apparatus. The yield of n-butyl lactate determined by gas chromatrography was 40.1% based on dihydroxyacetone.

## Example 3

A stock solution (A) was made combining:
n-butanol 100ml
dihydroxyacetone dimer 11.25g
diethylene glycol dimethyl ether 20.7g

The mixture was warmed slightly to assist solution of dihydroxyacetone dimer.

## Example 3a

Into a 2ml vial equipped with septum was added 1ml of stock solution (A) together with 0.01g of chromium (III) acetylacetonate. The vial was sealed and placed in an oven at 115°C. Molar yields of n-butyl lactate were 12.6% after 1 hour and 53.4% after 66 hours.

## Example 3b

Into a 2ml vial equipped with septum was added 1ml of stock solution (A) together with 0.01g of zirconocene dichloride. The vial was sealed and placed in an oven at 115°C. Molar yields of n-butyl lactate were 28.4% after 1 hour and 72.6% after 66 hours

## Example 3c

Same procedure as 3a except 0.01g of zirconyl chloride octahydrate was employed as catalyst. Yield of butyl lactate was 68.7% after 15 hours.

## Example 3d

Same procedure as 3a except 0.01g of vanadyl acetylacetonate was employed as catalyst. Yield of butyl lactate was 13.4% after 15 hours.

## Example 3e

Same procedure as 3a except 0.1g of niobium pentoxide was employed as catalyst. Yield of butyl lactate was 2.2% after 15 hours.

## Example 3f

Same procedure as 3a except 0.01g of ruthenium trichloride was employed as catalyst. Yield of butyl lactate was 18.7% after 15 hours.

## Example 3g

Same procedure as 3a except of 0.01g of ammonium tetrachloropalladate was employed as catalyst. Yield of butyl lactate was 19.3% after 15 hours.

## Example 3h

Same procedure as 3a except 0.01g of rhodium trichloride hydrate was employed as catalyst. Yield of butyl lactate was 16.8% after 15 hours

## Example 3i

Same procedure as 3a except 0.01g of zirconium tetrachloride was employed as catalyst. Yield of butyl lactate was 73.5% after 15 hours.

## Example 3j

Same procedure as 3a except 0.01g of zirconium nitrate was employed as catalyst. Yield of butyl lactate was 6.5% after 15 hours.

## Example 3k

Same procedure as 3a except 0.01g of chromium (III) acetate was employed as catalyst. Yield of butyl lactate was 32.4% after 15 hours.

## Example 3l

Same procedure as 3a except 0.01g of chromium (III) nitrate nonahydrate was employed as catalyst. Yield of butyl lactate was 34.0% after 15 hours.

## Example 3m

Same procedure as 3a except 0.01g of chromium trichloride hexahydrate was employed as catalyst. Yield of butyl lactate was 81.6% after 15 hours.

## Example 3n

Same procedure as 3a except 0.01g of molybdenum pentachloride was employed as catalyst. Yield of butyl lactate was 39.6% after 15 hours.

## Example 4

A stock solution (B) was made by combining ethanol 100ml dihydroxyacetone dimer 11.25g diethylene glycol dimethyl ether 20.7g

The mixture was warmed slightly to assist solution of dihydroxyacetone dimer.

## Example 4a

Into a glass lined pressure vessel was added 10ml of stock solution (B) together with 0.1g of zirconocene dichloride. The vessel was purged with nitrogen, sealed and heated to 150°C for 15 hours. After cooling to ambient temperature the vessel was opened and the contents analysed for ethyl lactate by gas chromatography. A 32.3% yield of ethyl lactate based on dihydroxyacetone was obtained.

## Example 4b

Into a glass lined pressure vessel was added 10ml of stock solution (B) together with 0.1g of chromium (III) acetylacetonate. The vessel was purged with nitrogen, sealed and heated to 150°C for 15 hours. After cooling to ambient temperature the vessel was opened and the contents analysed for ethyl lactate by gas chromatography. A 49.1% yield of ethyl lactate based on dihydroxyacetone was obtained.

## Example 5

A solution was made up containing 20ml ethanol, 4g dihydroxyacetone, 1.18g chromium trichloride hexahydrate and 5.84g of diethylene glycol dimethyl ether. Some warming was required in order to dissolve all of the dihydroxyacetone. The solution was heated to 115°C in a pressure vessel for 16.5 hours. After cooling to ambient temperature and pressure it was analysed for ethyl lactate and lactic acid by HPLC. The yield of ethyl lactate was 62.3% and the yield of lactic acid was 13.3%.

Example 6

Into a 250ml flask was added 10.02g dihydroxyacetone, 0.53g of Tilcom TL aqueous titanium lactate solution and 150.38g water. The mixture was heated under reflux under a nitrogen atmosphere for 7 hours. The yield of lactic acid was 4.5%.

Claims

1. A process for the production of alpha-hydroxy carboxylic compounds said process comprising reacting

   a) a carbonyl compound having at least one hydroxyl group alpha to the carbonyl function with
   b) a hydroxy compound of the formula R³OH wherein R³ is H, an alkyl group, an alkenyl group, a cycloalkyl group or an aryl group at elevated temperature in the presence of a transition metal compound as catalyst.

2. A process according to Claim 1 wherein the carbonyl compound having at least one hydroxyl group alpha to the carbonyl function has the generic formula

   $$HO.H_2C.CO.C(R)(R^1)(R^2) \qquad (I)$$

   wherein each of R, R¹ and R² may be the same or different groups selected from hydrogen, hydroxyl, alkyl, cycloalkyl, alkaryl, aryl, aralkyl, halo, nitro, nitrile and a sugar residue.

3. A process according to Claim 2 wherein if any of R, R¹ or R²:

   (i) or all three taken together represent an alkyl group, it is a straight or branched chain $C_1$-$C_{10}$ alkyl group;
   (ii) or all three taken together represent an aryl group, it has one or more aromatic nuclei whether or not forming fused ring structures;
   (iii) represent a halo group, it is a chloro or a bromo group;
   or,
   (iv) represent a sugar residue, such residues being those derived from glucose, fructose or sucrose.

4. A process according to any of the preceding Claims wherein the carbonyl compound having at least one hydroxyl group alpha to the carbonyl function is 1,3-dihydroxyacetone.

5. A process according to any one of the preceding Claims wherein R³ is a $C_1$-$C_{10}$ hydrocarbyl group.

6. A process according to any one of the preceding Claims wherein the catalyst used is a transitional metal belonging to any one of the Groups III-VIII of the Periodic Table of Elements.

7. A process according to any one of the preceding Claims wherein the catalyst is a compound of a metal selected from titanium, zirconium, hafnium, vanadium, niobium, chromium, ruthenium and rhodium.

8. A process according to any one of the preceding Claims wherein the catalyst is used in an amount from 0.001 to 1 mole based on the weight of the reactant (a).

9. A process according to any one of the preceding Claims wherein the reaction is carried out under homogeneous conditions, using a soluble transition metal catalyst.

10. A process according to any one of the preceding Claims wherein 1,3-dihydroxyacetone is reacted with n-butanol in the presence of titanium isopropoxide or titanium lactate to form n-butyl lactate.